(19) Europäisches Patentamt

European Patent Office

Office européen des brevets

(11) **EP 1 062 979 A2**

(12) **EUROPÄISCHE PATENTANMELDUNG**

(43) Veröffentlichungstag:
    **27.12.2000  Patentblatt 2000/52**

(51) Int. Cl.⁷: **A61N 1/37**

(21) Anmeldenummer: **00250195.5**

(22) Anmeldetag: **23.06.2000**

(84) Benannte Vertragsstaaten:
    **AT BE CH CY DE DK ES FI FR GB GR IE IT LI LU
    MC NL PT SE**
    Benannte Erstreckungsstaaten:
    **AL LT LV MK RO SI**

(30) Priorität: **23.06.1999 DE 19929553**

(71) Anmelder:
    **BIOTRONIK Mess- und
    Therapiegeräte GmbH & Co
    Ingenieurbüro Berlin
    12359 Berlin (DE)**

(72) Erfinder: **Meier, Jan
    91080 Uttenreuth (DE)**

(74) Vertreter:
    **Eisenführ, Speiser & Partner
    Pacelliallee 43/45
    14195 Berlin (DE)**

(54) **Herzschrittmacher**

(57)    Um bei einem Herzschrittmacher mit mindestens einer im Bereich des Herzens (1) angeordneten Stimulationselektrode (2), die ausgehend von einem Impulsgenerator (3) Stimulationsimpulse an das Herz (1) abgibt und einer mit dem Impulsgenerator (3) verbundenen Steuereinheit (4) zur Steuerung der Stimulationsimpulsabgabe durch den Impulsgenerator (3), die eingangsseitig mit Sensormitteln (5) zur Kontrolle des Stimulationserfolges am Herzen (1) durch Impedanzmessung verbunden ist, einen Stimulationserfolg unmittelbar nach der Stimulationsimpulsabgabe zuverlässig zu detektieren, wird vorgeschlagen, daß die am Myokard (6) angeordneten Sensormittel (5) den myokardialen Impedanzverlauf während oder unmittelbar nach der Stimulationsimpulsabgabe und noch innerhalb der diastolischen Phase des Herzens (1) detektieren und die Steuereinheit (4) die Stimulationsimpulsabgabe des Impulsgenerators (3) unter Berücksichtigung von Impedanzänderungen im myokardialen Impedanzverlauf steuert.

Fig.1

**Beschreibung**

Die Erfindung betrifft einen Herzschrittmacher gemäß dem Oberbegriff des Anspruches 1.

Herzschrittmacher der hier interessierenden Art sind mit Sensormitteln für eine Impedanzmessung zur Kontrolle des Stimulationserfolges am Herzen (capture-detection) ausgestattet. Bekanntermaßen läßt der kardiale Impedanzverlauf Rückschlüsse auf diverse Herzfunktionen zu, insbesondere auf den Stimulationserfolg. Abhängig vom Stimulationserfolg ist damit insbesondere eine automatische Anpassung der erzeugten Stimulationsimpulse an die Stimulationsreizschwelle möglich. Die Stimulationsreizschwelle kennzeichnet diejenige Stimulationsamplitude, die erforderlich ist, um einen Stimulationserfolg, d.h. eine stimulierte Systole auszulösen. Da sich die Stimulationsreizschwelle infolge des Hormonspiegels, der Tageszeit, der körperlichen Aktivität etc, ständig ändert, ist die Stimulationsamplitude der zeitlichen Änderung der Stimulationsreizschwelle anzupassen. Wird beispielsweise durch einen ausbleibenden Stimulationserfolg nach Abgabe eines Stimulationsimpulses (infolge eines ausbleibenden Impedanzanstieges im Impedanzverlauf nach Abgabe des Stimulationsimpulses) eine unterschwellige Stimulation erkannt, so ist die Stimulationsamplitude zu erhöhen, um eine zuverlässige Funktion des Herzschrittmachers sicherzustellen.

Andererseits ist auch bei Betrieb des Herzschrittmachers ein minimaler Energiebedarf gefordert, um eine lange Lebensdauer der in dem Herzschrittmacher integrierten Batterie zu gewährleisten. Daher ist die Stimulationsamplitude nur soweit wie erforderlich, also über ein nur geringes Sicherheitsmarginal über der Stimulationsreizschwelle hinaus, zu erhöhen.

Allgemein bekannt sind neben einer Kontrolle des Stimulationserfolges anhand einer Impedanzmessung auch Methoden, die sich der Messung des durch den Stimulationsimpuls evozierten Potentials oder des QRS-Komplexes bedienen. Hierbei ist eine zuverlässige Erkennung des Stimulationserfolges jedoch frühestens 100 bis 130 Millisekunden nach Abgabe des Stimulationsimpulses durch den Herzschrittmacher und damit sehr zeitverzögert möglich, was kurzfristige korrigierende Maßnahmen in der Stimulationsimpulssteuerung erschwert.

Aus der US 5,766,230 ist ein Herzschrittmacher bekannt, bei dem die Kontrolle des Stimulationserfolges noch während der Abgabe des Stimulationsimpulses und damit sehr schnell erfolgt. Zu Ende des ansonsten rechteckförmigen Stimulationsimpulses fällt die Stimulationsamplitude bedingt durch einen plötzlich höheren, den Stimulationserfolg signalisierenden Stromfluß jäh ab, was eine Abfallkurve in der Impulsform bewirkt. Diese Abfallkurve in der Impulsform wird zur Erkennung des Stimulationserfolges herangezogen. Es ist hierbei jedoch von Nachteil, daß der Eintritt eines Stimulationserfolges nur zu erkennen ist, wenn sich die Impulsform schon vor Ende des Stimulationsimpulses ändert. Ansonsten ist ein Stimulationserfolg nicht erkennbar, obwohl er stattfindet. Wie Simulationen zeigen, führen Stimulationsimputsamplituden nahe der Stimulationsreizschwelle erst mit einer Verzögerung von bis zu 3 Millisekunden zum Aktivieren des Stimulationserfolges. Zu diesem Zeitpunkt ist der Stimulationsimpuls bereits abgegeben. Ein Stimulationserfolg ist folglich nur dann zuverlässig zu erkennen, wenn die Impulsamplituden deutlich über der Stimulationsreizschwelle liegen, weil die Verzögerung hier noch innerhalb der Impulsabgabe liegt. Die Detektion eines Stimulationserfolges erfolgt mit dieser Methode also nicht zuverlässig. Werden zur Sicherstellung einer zuverlässigeren Detektierbarkeit stets Stimulationsimpulse mit überhohen Amplituden erzeugt, so entsteht wiederum ein zu hoher Energiebedarf.

Aus der US 5,843,137 ist ein gattungsgemäßer Herzschrittmacher bekannt, der sich einer intrakardialen Impedanzmessung zur Kontrolle des Stimulationserfolges bedient. Der Herzschrittmacher besteht im wesentlichen aus einem Impulsgenerator zur Erzeugung von Stimulationsimpulsen, die von einer Steuereinheit gesteuert über eine Elektrode abgegeben werden sowie Sensormitteln zum Messen von physiologischen Parametern die im Zusammenhang mit der Herzfunktion stehen und mit denen der Stimulationserfolg über die Steuereinheit auswertbar ist. Die Sensormittel nehmen die Impedanzänderungen und andere physiologische Werte auf, die im Zusammenhang mit einer stimulierten Systole stehen. Die Steuereinheit ist in der Lage aus den Eingangsdaten zwischen einer natürlichen und einer stimulierten Systole zu unterscheiden. Damit kann die Steuereinheit unter anderem eine Nachführung der Stimulationsamplituden an die Stimulationsreizschwelle in Abhängigkeit vom Stimulationserfolg vornehmen. Die Messung der kardialen Impedanz erfolgt intrakardial. Zu diesem Zwecke wird die Impedanz zwischen zwei dem Herzschrittmacher zugeordneten Elektroden gemessen (Seite 12, Zeilen 7 bis 9). Als Elektroden kommen hier das Gehäuse des Herzschrittmachers selbst, im Ventrikel oder Atrium eingeführte Elektrodenleitungen sowie Ringelektroden zum Einsatz. Die Messung erfolgt gewöhnlich getaktet, d.h. in äquidistanten Zeitabständen wird die eine Elektrode mit einer Meßspannung beaufschlagt und es wird der Strom gemessen, der zwischen beiden Elektroden fließt. Die Impedanz ergibt sich in bekannter Weise als Quotient zwischen Meßspannung und Strom.

Ein derartiger Herzschrittmacher ist zwar in der Lage, grundsätzlich einen Stimulationserfolg zu erkennen; es ist jedoch von Nachteil, daß die Erkennung erst nach Ablauf einer größeren Zeitspanne nach Stimulationsimpulsabgabe möglich ist. Dies ist durch das Wesen der intrakardialen Impedanzmessung bedingt. Der große Zeitabstand verhindert es beispielsweise noch rechtzeitig eine korrigierende Maßnahme - wie einen Sicherheits-Stimulationsimpuls - zu erzeugen, falls der Stimulationserfolg ausbleibt. Wenn ein Sicherheits-Stimulationsimpuls trotzdem abgegeben wird, so kann dadurch der Herzzyklus, d.h. die zeitliche Abfolge der diastolischen und systolischen Phase des Herzens, erheblich gestört werden.

Es ist daher die Aufgabe der vorliegenden Erfindung, einen gattungsgemäßen Herzschrittmacher dahingehend weiter zu verbessern, daß der Stimulationserfolg unmittelbar nach der Stimulationsimpulsabgabe zuverlässig detektierbar ist.

Die Aufgabe wird ausgehend von einem Herzschrittmacher gemäß dem Oberbegriff des Anspruches 1 in Verbindung mit dessen kennzeichnenden Merkmalen gelöst. Die nachfolgenden Unteransprüche geben vorteilhafte Weiterbildungen der Erfindung an.

Die Erfindung schließt die technische Lehre ein, daß für eine zuverlässige Detektion des Stimulationserfolges unmittelbar nach der Stimulationsimpulsabgabe die Sensormittel am Myokard angeordnet sind und somit den myokardialen Impedanzverlauf unmittelbar nach der Stimulationsimpulsabgabe und noch innerhalb der diastolischen Phase des Herzens detektieren, wobei die Steuereinheit die Stimulationsimpulsabgabe des Impulsgenerators unter Berücksichtigung von Impedanzänderungen im myokardialen Impedanzverlauf steuert.

Der Erfindung liegt die Erkenntnis zu Grunde, daß der Stimulationserfolg sich im Verlauf der myokardialen Impedanz widerspiegelt, und zwar während oder in unmittelbarer Folge der Stimulationsimpulsabgabe durch den Herzschrittmacher (zirka innerhalb 2 bis 10 Millisekunden); genauer gesagt in einer Phase, in der das Herz noch nicht mechanisch auf den Stimulationsimpuls reagiert hat. Die mechanische Reaktionszeit des Herzens beträgt zirka 50 bis 70 Millisekunden. Im Gegensatz dazu ist ein Stimulationserfolg bei bekannter Messung der intrakardialen Impedanz erst deutlich später möglich. Die Erscheinung der kurzfristigen Detektierbarkeit anhand der myokardialen Impedanz beruht auf elektro-physiologischen Vorgängen im Myokard. Die Veränderungen im Impedanzverlauf werden überwiegend hervorgerufen durch Änderungen in den Membraneigenschaften und Ionenverschiebungen zwischen extrazellulärem Raum und dem Inneren der Herzmuskelzelle. Bei Stimulationsimpulsabgabe nimmt die Gewebeimpedanz durch Öffnen der $Na^+$ - Kanäle und damit einhergehendem Leitfähigkeitsanstieg der Zellmembranen kurzfristig für die Dauer von zirka 1 bis 2 Millisekunden stark ab. Die Abnahme erfolgt etwa um den Faktor 3. Dieses über die Sensormittel eindeutig erfaßbare Ereignis kennzeichnet den darauffolgenden Eintritt eines Stimulationserfolges, also der Systole des Herzens. Somit ist es möglich den Eintritt oder das Ausbleiben eines Stimulationserfolges unmittelbar nach der Stimulationsimpulsabgabe zuverlässig zu Erkennen.

Ein besonderer Vorteil der ultraschnellen Detektion eines Stimulationserfolges ist, daß neben einer herkömmlichen Nachführung der Stimulationsamplituden an die Stimulationsreizschwelle in Abhängigkeit vom Stimulationserfolg auch eine rechtzeitige Generierbarkeit eines Sicherheits-Stimulationsimpulses bei ausbleibendem Stimulationserfolg erfolgen kann. Der Sicherheits-Stimulationsimpuls kann somit für den Patienten unbemerkt abgegeben werden und stört nicht den Herzzyklus. Insoweit ist eine Nachführung der Stimulationsamplituden möglich, ohne daß der Herzschrittmacherträger wie bei vorbekannten Methoden für eine Kontrolle des Stimulationserfolges an "Aussetzern" leidet. Weiterhin kann ein ansonsten üblicher Tresholdtest durch den Arzt entfallen.

Um einen Sicherheits-Stimulationsimpuls abzugeben, kann vorzugsweise die Steuereinheit des Herzschrittmachers bei einem infolge unterschwelliger Stimulation ausbleibendem Stimulationserfolg des Herzens den Sicherheits-Stimulationsimpuls auslösen. Ebenso können hierfür separate elektronische Mittel vorgesehen werden.

Vorzugsweise sind die Sensormittel in der Stimulationselektrode integriert. Damit sind Sensormittel und Stimulationselektroden funktionsintegriert untergebracht. Die Stimulationselektrode kann insbesondere nach Art einer fraktalen Elektrode ausgebildet sein. Mittels einer fraktalen Elektrode sind Änderungen im myokardialen Impedanzverlauf bereits wenige Millisekunden nach der Stimulationsimpulsabgabe wahrnehmbar. Als Blankingzeit sind lediglich bis zu 2 Millisekunden erforderlich. Weiterhin ist es auch denkbar, die Sensormittel und verschieden ausgebildete Stimulationselektroden extra an den Herzschrittmacher anzuschließen.

Es ist vorteilhaft, wenn die Messung der myokardialen Impedanz nach der StromSpannungs-Methode erfolgt. Zu diesem Zwecke bestehen die Sensormittel als Elektrodensatz vorzugsweise aus mindestens zwei beabstandet auf dem Myokard angeordneten Kontakten, wovon ein Kontakt vom Impulsgenerator mit einem Wechselstrom beaufschlagt ist und der andere als Meßelektrode dienende Kontakt zur Ermittlung der myokardialen Impedanz mit der Steuereinheit verbunden ist. Um einen unterhalb von ca. 1 Kiloherz auftretenden störenden Einfluß der Elektrodenpolarisation weitestgehend auszuschließen, können gegebenenfalls auch mehrere nebeneinanderliegende Elektrodensätze gewählt werden.

Eine weitere die Erfindung verbessernde Maßnahme sieht vor, daß zur Ermittlung einer Impedanzänderung im myokardialen Impedanzverlauf eine Messung der myokardialen Impedanz unmittelbar vor der Stimulationsimpulsabgabe erfolgt. Damit werden definierte Detektionszeitfenster geschaffen, die einen Vergleich von Impedanzänderungen bei minimalem Energiebedarf ermöglichen. Der Energiebedarf ist trotz des durch Anwendung der Strom-Spannungs-Methode zur myokardialen Impedanzmessung erforderlichen Meßstroms somit gering.

Nach einer weiteren Verbesserungsmaßnahme der Erfindung kann zur Feststellung des Maßes einer Überschreitung der Stimulationsreizschwelle die Steuereinheit den zeitlichen Abstand vom vorangegangenen überschwelligen Stimulationsimpuls ermitteln. Somit ist die Nachführfunktion der Stimulationsimpulse an die Stimulationsreizschwelle effektiv optimierbar.

Eine andere verbessernde Maßnahme sieht vor, daß durch die Steuereinheit neben der Kontrolle des Stimulationserfolges am Herzen auch eine Kontrolle von natürlichen Herzaktionen erfolgt. Zur Detektion von natürlichen Herzaktionen kann die Steuereinheit vorzugsweise den myokardialen Impedanzverlauf kontinuierlich überwachen und eine natürliche Herzaktion anhand eines kurzzeitigen Impedanzabfalls im myokardialen Impedanzverlauf ohne vorherige Stimulationsimpulsabgabe ermitteln. Diese Methode ist in vorteilhafter Weise unempfindlich gegenüber beispielsweise Myopotentialen.

Um bei der kontinuierlichen Überwachung eine zuverlässige Detektion von Herzaktionen unter mäßigen Energiebedarf sicherzustellen, kann der myokardiale Impedanzverlauf mit einer Samplingrate von vorzugsweise zirka 500 Hz erfaßt werden. Dies kann über den gesamten Zeitverlauf oder - zur weiteren Energieersparnis - in vorher definierten Zeitfenstern durchgeführt werden.

Weitere die Erfindung verbessernde Maßnahmen werden nachstehend gemeinsam mit der Beschreibung eines bevorzugten Ausführungsbeispieles der Erfindung anhand der Figuren näher dargestellt. Es zeigt:

- Figur 1    Ein prinzipielles Blockschaltbild eines Herzschrittmachers,

- Figur 2    ein Ersatzschaltbild für das Herzmuskelgewebe zur Messung der myokardialen Impedanz und

- Figur 3    ein Diagramm des zeitlichen Verlaufs einer vom Herzschrittmacher abgegebenen Impulsfolge während eines Meßzyklus.

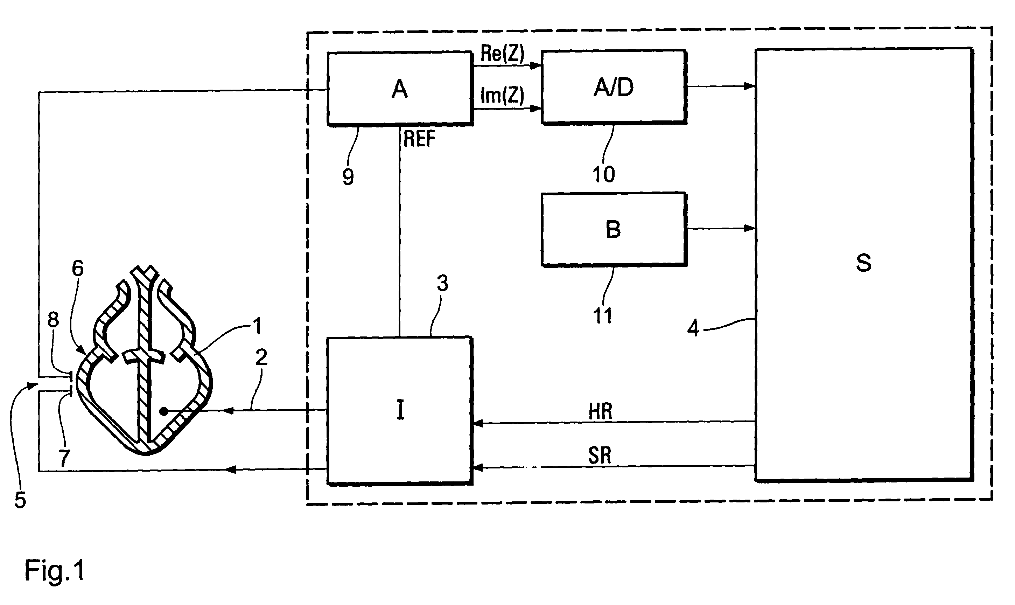

Der Herzschrittmacher gemäß Figur 1 ist als aktivitätsgesteuerter Herzschrittmacher ausgeführt und vermag damit belastungsabhängige Aktivitätsgrößen seines Trägers - wie Bewegung oder tageszyklische Herzrate - zu erkennen und in Abhängigkeit davon die adaptive Herzrate einzustellen.

Zu diesem Zwecke ist im rechten Ventrikel des Herzen 1 eine Stimulationselektrode 2 eingebracht. Die Stimulationselektrode 2 dient zur Abgabe von Stimulationsimpulsen, die von einem Impulsgenerator 3 gesteuert abgegeben werden. Die Steuerung des Impulsgenerators 3 erfolgt über eine Steuereinheit 4, die der von ihr ermittelten Herzrate (HR) entsprechende Steuersignale ausgibt. Weiterhin sind Sensormittel 5 zur Detektion der Impedanz des Myokard 6 hier zur besseren Veranschaulichung des Arbeitsprinzips separat vorgesehen. (Vorzugsweise sind die Sensormittel 5 jedoch in der Stimulationselektrode 2 direkt integriert.) Der Wert der gemessenen myokardialen Impedanz wird in der Steuereinheit 4 zur Beeinflussung der Herzrate, d.h. letztlich zur Abgabe der Stimulationsimpulse, verwendet. Die Sensormittel 5 umfassen - in der dargestellten Ausführungsform - zwei an ihrer Oberfläche vergoldete Kontakte 7 und 8, die auf dem Myokard 6 an geeigneter Stelle aufgesetzt sind und bevorzugt eine fraktale Geometrie besitzen. Der erste Kontakt 7 ist mit dem Impulsgenerator 3 verbunden, der Meßimpulse gesteuert mit einer von der Steuereinheit 4 bestimmten Samplingrate (SR) als Meßstrom abgibt. Die Meßimpulse mit der Samplingrate (SR) werden zum Zwecke einer Energieersparnis nur während definierter Zeitfenster abgegeben. Alternativ können die Meßimpulse auch über die Stimulationselektrode 2 abgegeben werden. Der Kontakt 7 ist dann vorteilhafterweise entbehrlich.

Der in das Myokard 6 mehrere Millimeter tief eindringende Meßstrom von zirka 10 Mikroampere (effektiv) wird durch die Steuereinheit 4 konstant gehalten, um den Meßfehler zu minimieren. Den Spannungsabfall über dem Gewebe - dessen Ursache an späterer Stelle beschrieben wird - greift der als Meßelektrode dienende zweite Kontakt 8 als Meßspannung ab. Der Kontakt 8 ist mit einer Impedanz-Analysatoreinheit 9 mit hohem Eingangswiderstand verbunden, die zunächst das Meßsignal verstärkt.

Die Impedanz-Analysatoreinheit 9 ist in Lock-In-Technik ausgeführt und zerlegt die Meßspannung in einen Realteil, der in Phase mit dem Meßstrom ist, und einem Imaginärteil, der um 90° gegen die Meßstromphasen versetzt ist. Hierzu ist der Impulsgenerator 3 an einen Referenzsignaleingang (REF) am Impedanz-Analysatoreinheit 9 angeschlossen. Die gewonnenen Meßspannungskomponenten sind direkt proportional dem Realteil Re(Z) und dem Imaginärteil Im(Z) der Impedanz Z = U/I bei konstantem Meßstrom. Re(Z) und Im(Z) bilden die Ausgangssignale der Impedanz-Analysatoreinheit 9. Die Steuereinheit 4 übernimmt über einen Multiplexer mit Analog/Digital-Umsetzer 10 die Ausgangssignale. Aus dem Realteil Re(Z) und dem Imaginärteil Im(Z) der Impedanz Z errechnet die Steuereinheit 4 den Betrag der Impedanz Z sowie den Phasenwinkel nach folgender Formel:

$$|Z| = \mathrm{sqr}(\mathrm{Re}^2(Z) + \mathrm{Im}^2(Z))$$

$$\mathrm{phi} = \arctan \mathrm{Im}(Z) / \mathrm{Re}(Z)$$

Die insoweit aufgearbeiteten Meßwerte verkörpern den myokardialen Impedanzverlauf, der von der Steuereinheit 4 als eine Aktivitätsgröße (A) weiterverarbeitet wird.

Der myokardiale Impedanzverlauf wird durch die Steuereinheit 4 hinsichtlich langsamer Veränderungen mittels Differenzation analysiert. Dadurch paßt sich der Herzschrittmacher an langsam veränderliche Randbedingungen, wie Lebensgewohnheit, Alter oder Medikation an. Zur Verbesserung des Schrittmacherverhaltens bei kurzfristiger physischer Belastung ist weiterhin ein Bewegungssensor 11 vorgesehen, dessen Ausgangswert ebenfalls als Aktivitätsgröße (A) von der Steuereinheit 4 verarbeitet wird. Der Steuereinheit 4 ist zur Stimulation des Herzens 1 der Impulsgenerator 3 nachgeschaltet, der gesteuert durch die von der Steuereinheit ermittelte Herzrate (HR) Stimulationsimpulse generiert. Dabei arbeitet der Herzschrittmacher nach dem Anforderungsprinzip, d.h. der Herzschrittmacher stimuliert das Herz 1 nur dann, wenn innerhalb einer bestimmten Wartezeit nach einer vorangegangenen Systole des Herzens 1 keine weitere durch die Sensormittel 5 detektierte Systole des Herzens 1 durch eine natürliche Stimulation stattfindet.

Durch Analyse des myokardialen Impedanzverlaufes mittels der Steuereinheit 4 nach der Stimulationsimpulsabgabe durch die Stimulationselektrode 2, ist der Eintritt eines Stimulationserfolges unmittelbar nach der Stimulationsimpulsabgabe detektierbar. Somit ist die Steuereinheit 4 in der Lage bei ausbleibendem Stimulationserfolg nach einer Stimulationsimpulsabgabe aufgrund einer unterschwelligen Stimulation (die auf einen kurzfristigen Anstieg der Stimulationsreizschwelle aufgrund veränderter Aktivitätsgrößen A des Herzschrittmacherträgers beruhen kann) noch rechtzeitig einen Sicherheits-Stimulationsimpuls zu initiieren, den der nachgeschaltete Impulsgenerator 3 generiert und der durch die Stimulationselektrode 2 abgegeben wird. Der Sicherheits-Stimulationsimpuls ist somit bei Bedarf innerhalb kürzester Zeit abgebbar, so daß der natürliche Herzzyklus hierdurch nicht gestört wird.

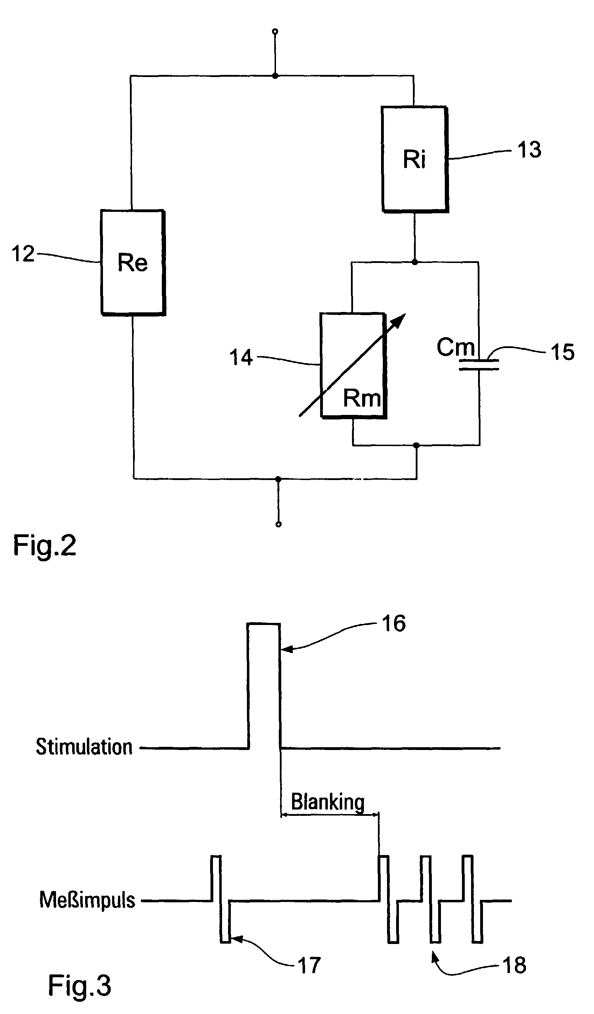

Die kurzfristige Detektierbarkeit eines Stimulationserfolges beruht auf einen anhand der Figur 2 beschreibbaren Effekts des Impedanzverhaltens im Myokard 6. Das hier gezeigte Ersatzschaltbild in Form eines linearen Netzwerkes repräsentiert mit seinem Widerstand (Re) 12 die Impedanz der extrazellulären Flüssigkeit. Der parallel dazu geschaltete Widerstand (Ri) 13 beschreibt die Impedanz der intrazellulären Strukturen und Flüssigkeit. Der nachgeschaltete Widerstand (Rm) 14 steht für den ohmschen Anteil der Membranimpedanz der Myokardzelle und der parallel hierzu geschaltete Kondensator (Cm) 15 steht für die Membrankapazität der Myokardzelle. Bei niedrigen Frequenzen bis 10 KHz wird die Gewebeimpedanz von Re bestimmt. Der Membranwiderstand Rm ist sehr hoch.

Ebenso ist der Blindwiderstand von Cm sehr groß gegenüber Re. Der Strom fließt also im wesentlichen über Re, die Impedanz ist praktisch real und gibt den Widerstand der extrazellulären Leitungswege wieder, der im wesentlichen bestimmt ist durch die spezifische Leitfähigkeit der elektrolytischen Flüssigkeit. Bei hohen Frequenzen über 1 MHz ist Ri der bestimmende Faktor, da der Membranwiderstand Rm von der Membrankapazität Cm überbrückt wird. Dabei ist Ri zirka das 0,3-fache von Re. Die größten Veränderungen im Impedanzspektrum sind überwiegend hervorgerufen durch Änderungen in den Membraneigenschaften und lonenverschiebungen zwischen extrazellulärem Raum in dem Inneren der Zellen. Die elektrische Impedanz gibt somit Auskunft über Vorgänge im Gewebe. Eine Systole des Herzens 1 nach erfolgtem Stimulationsimpuls kündigt sich durch ein kurzfristiges Öffnen der Na$^+$ - Kanäle von zirka 1 bis 2 Millisekunden und eine mit dieser lonenverschiebung einhergehende starke Abnahme der Impedanz an. Über die Erfassung dieser Impedanzabnahme ist der Stimulationserfolg unmittelbar nach Stimulationsimpulsabgabe detektierbar.

Gemäß Figur 3 erfolgt die Detektion eines Stimulationsimpulses 16 unmittelbar nach Ablauf der Blankingzeit durch Meßimpulse 18; zu einem Zeitpunkt also, bei dem sich das Herz 1 noch in seiner diastolischen Phase befindet, eine mechanische Arbeit also noch nicht stattgefunden hat. Die mechanische Arbeit (Systole) setzt erst nach zirka 50 bis 70 Millisekunden ein. Da ein Stimulationserfolg einen kurzen Dip im Impedanzverlauf erzeugt, ist der Stimulationserfolg durch die Meßimpulse 18 erkennbar. Zum Vergleich wird auch die Impedanz unmittelbar vor Abgabe des Stimulationsimpulses 16 durch einen Meßimpuls 17 ermittelt.

In Ergebnis der insoweit kurzfristigen Erkennbarkeit eines Stimulationsmißerfolges ist es vorzugsweise möglich einen rechtzeitigen und damit physiologisch unschädlichen Sicherheits-Stimulationsimpuls abzugeben. Weiterhin enthält der detektierte Impedanzabfall auch Informationen darüber, wie weit die aktuelle Stimulationsamplitude über dem Stimulationsschwellwert liegt. Diese Information nutzt die Steuereinheit 4 zur automatischen Schwellwertanpassung der Stimulationsimpulse. Schließlich eignet sich eine kontinuierliche Detektion des myokardialen Impedanzverlaufes auch zur Erkennung natürlicher Systolen des Herzens 1, da ja auch hierbei ein kurzfristiges Abfallen der Impedanz stattfindet. Es erfolgt eine dementsprechende Berücksichtigung in der Stimulationsimpulsabgabe durch die Steuereinheit 4.

Zur Minimierung der Blankingzeit wird - in Abweichung zu dem das Arbeitsprinzip veranschaulichende Ausführungsbeispiel - die Verwendung einer einzigen fraktalen Elektrode für die Stimulationsimpulsabgabe sowie die Impedanzmessung empfohlen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht. Insbesondere ist die Erfindung hinsichtlich der Aktivitätsgrößen (A) nicht auf bestimmte biologische Größen beschränkt. Vielmehr können alle Größen herangezogen werden, die eine Abhängigkeit von der physischen oder psychischen Belastung des Herzschrittmacherträgers zeigen. Dasselbe gilt auch für die beispielhaft angegebene Elektrodenanordnung zur Stimulation und Sensorik.

**Patentansprüche**

1. Herzschrittmacher mit mindestens einer im Bereich des Herzens (1) angeordneten Stimulationselektrode (2), die ausgehend von einem Impulsgenerator (3) Stimulationsimpulse an das Herz (1) abgibt und einer mit dem Impulsgenerator (3) verbundenen Steuereinheit (4) zur Steuerung der Stimulationsimpulsabgabe durch den Impulsgenerator (3), die eingangsseitig mit Sensormitteln (5) zur Kontrolle des Stimulationserfolges am Herzen (1) durch Impedanzmessung verbunden ist, dadurch gekennzeichnet, daß die am Myokard (6) angeordneten Sensormittel (5) ausgebildet sind, den myokardialen Impedanzverlauf während oder unmittelbar nach der Stimulationsimpulsabgabe und noch innerhalb der diastolischen Phase des Herzens (1) zu detektieren und die Steuereinheit (4) die Stimulationsimpulsabgabe des Impulsgenerators (3) unter Berücksichtigung von Impedanzänderungen im myokardialen Impedanzverlauf anzusteuern.

2. Herzschrittmacher nach Anspruch 1, dadurch gekennzeichnet, daß die Sensormittel (5) ausgebildet sind, eine kurzfristige Impedanzverminderung zu detektieren.

3. Herzschrittmacher nach Anspruch 1 oder 2, dadurch gekennzeichnet, daß die Steuereinheit (4) mit den Sensormitteln (5) verbunden und ausgebildet ist, im Falle des Ausbleibens einer kurzfristigen Impedanzverminderung innerhalb eines vorgebbaren Zeitraumes nach Abgabe eines Stimulationsimpulses die Abgabe eines Sicherheits-Stimulationsimpulses auszulösen.

4. Herzschrittmacher nach einem der Ansprüche 1 bis 3, dadurch gekennzeichnet, daß die Stimulationselektrode (2) in die Sensormittel (5) integriert ist.

5. Herzschrittmacher nach Anspruch 4, dadurch gekennzeichnet, daß die Stimulationselektrode (2) nach Art einer fraktalen Elektrode ausgebildet ist.

6. Herzschrittmacher nach einem der vorhergehenden Ansprüche, dadurch gekennzeichnet, daß zur Messung der myokardialen Impedanz nach der Strom-Spannungs-Methode die Sensormittel (5) mindestens einen aus zwei beabstandet auf dem Myokard (6) angeordneten Kontakten (7, 8) bestehenden Elektrodensatz umfassen, wovon ein Kontakt (7) vom Impulsgenerator (3) mit Meßimpulsen beaufschlagt ist und der andere als Meßelektrode dienende Kontakt (8) zur Ermittlung der myokardialen Impedanz zur Steuereinheit (4) geführt ist.

7. Herzschrittmacher nach Anspruch 6, dadurch gekennzeichnet, daß zur Messung der myokardialen Impedanz nach der Strom-Spannungsmethode einer der beiden Kontakte (7,8) der Sensormittel (5) durch die Stimulationselektrode (2) ersetzt ist.

8. Herzschrittmacher nach einem der vorhergehenden Ansprüche, dadurch gekennzeichnet, daß mindestens einer der beiden Kontakte (7, 8) eine fraktale Geometrie aufweist.

9. Herzschrittmacher nach einem der vorhergehenden Ansprüche, dadurch gekennzeichnet, daß eine Messung des myokardialen Impedanzverlaufes 2 bis 10 Millisekunden nach der Stimulationsimpulsabgabe erfolgt.

10. Herzschrittmacher nach einem der vorhergehenden Ansprüche, dadurch gekennzeichnet, daß zur Ermittlung einer Impedanzänderung im myokardialen Impedanzverlauf eine Messung der myokardialen Impedanz unmittelbar vor der Stimulationsimpulsabgabe erfolgt.

11. Herzschrittmacher nach einem der vorhergehenden Ansprüche, dadurch gekennzeichnet, daß zur Feststellung des Maßes einer Überschreitung der Stimulationsreizschwelle die Steuereinheit (4) den zeitlichen Abstand vom vorangegangenen überschwelligen Stimulationsimpuls ermittelt.

12. Herzschrittmacher nach einem der vorhergehenden Ansprüche, dadurch gekennzeichnet, daß die Steuereinheit (4) bei einem infolge unterschwelliger Stimulation ausbleibendem Stimulationserfolg des Herzens (1) den Impulsgenerator (3) zur Abgabe eines Sicherheits-Stimulationsimpuls aussteuert.

13. Herzschrittmacher nach einem der vorhergehenden Ansprüche, dadurch gekennzeichnet, daß durch die Steuereinheit (4) neben der Kontrolle des Stimulationserfolges am Herzen (1) auch eine Kontrolle von natürlichen Herzaktionen erfolgt.

14. Herzschrittmacher nach Anspruch 13, dadurch gekennzeichnet, daß zur Detektion von natürlichen Herzaktionen die Steuereinheit (4) den myokardialen Impedanzverlauf kontinuierlich überwacht und eine natürliche Herzaktion anhand eines kurzzeitigen Impedanzabfalls im myokardialen Impedanzverlauf ohne vorherige Stimulationsimpulsabgabe ermittelt.

**15.** Herzschrittmacher nach einem der vorstehenden Ansprüche, dadurch gekennzeichnet, daß die vom Impulsgenerator (3) an einem Kontakt (7) abgegebenen Meßimpulse einer Samplingrate von größergleich 500 Hz aufweisen.

**16.** Herzschrittmacher nach Anspruch 15, dadurch gekennzeichnet, daß die Meßimpulse nur während vorgegebener Zeitfenster dem myokardialen Kontakt (7) zugeführt werden.

**17.** Herzschrittmacher nach einem der vorhergehenden Ansprüche, dadurch gekennzeichnet, daß zur Detektion physischer Belastungen ein Bewegungssensor (11) vorgesehen ist, dessen Ausgang im Ruhezustand des Herzschrittmacherträgers einen ersten Zustand und in Bewegung des Herzschrittmacherträgers einen zweiten Zustand einnimmt, wobei der Bewegungssensor (11) zur Beeinflussung der Stimulationsimpulsabgabe mit der Steuereinheit (4) eingangsseitig verbunden ist.

Fig.1

Fig.2

Fig.3